(19) Europäisches Patentamt

European Patent Office

Office européen des brevets

(11) **EP 1 618 833 A1**

(12) **EUROPEAN PATENT APPLICATION**

(43) Date of publication:
**25.01.2006 Bulletin 2006/04**

(51) Int Cl.:
**A61B 1/05** *(2006.01)* **H04N 5/217** *(2006.01)*

(21) Application number: **05015464.0**

(22) Date of filing: **15.07.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **20.07.2004 JP 2004212091**

(71) Applicant: **Olympus Corporation**
**Shibuya-ku, Tokyo (JP)**

(72) Inventors:
• **Sekimoto, Tomomi,**
  **Olympus Intell.Property**
  **Hachioji-shi**
  **Tokyo, 192-8512 (JP)**
• **Azuma, Motoo,**
  **Olympus Intell.Property**
  **Hachioji-shi**
  **Tokyo, 192-8512 (JP)**

(74) Representative: **Winter, Brandl, Fürniss, Hübner Röss, Kaiser,**
**Polte Partnerschaft Patent- und Rechtsanwaltskanzlei**
**Alois-Steinecker-Strasse 22**
**85354 Freising (DE)**

(54) **In vivo image pickup device and in vivo image pickup system**

(57)    An in vivo image pickup device (1) that is inserted in the body cavity and generates and sends an image signal of a subject, and includes an image pickup unit (11) that has a plurality of pixels with arrays on a light receiving surface thereof and converts a subject image formed on the light receiving surface into the image signal, a defect correcting circuit (12) that corrects the image signal of a defective pixel of the image pickup unit, a compressing circuit (13) that compresses the image signal from the defect correcting circuit, and a sending circuit (14) that sends the compressed image signal. Before the compressing circuit compresses the image signal, the defective pixel is corrected, thereby preventing the danger of harmful affection to a normal pixel from the defective pixel.

## FIG.1

1 : IN VIVO IMAGE PICKUP DEVICE

2 : RECEPTION PROCESSING DEVICE

EP 1 618 833 A1

**Description**

BACKGROUND OF THE INVENTION

1. Field of the Invention

The present invention relates to an in vivo image pickup device and an in vivo image pickup system, and more particularly, to an in vivo image pickup device and an in vivo image pickup system for detecting and correcting a defective pixel by a simple method.

2. Description of the Related Art

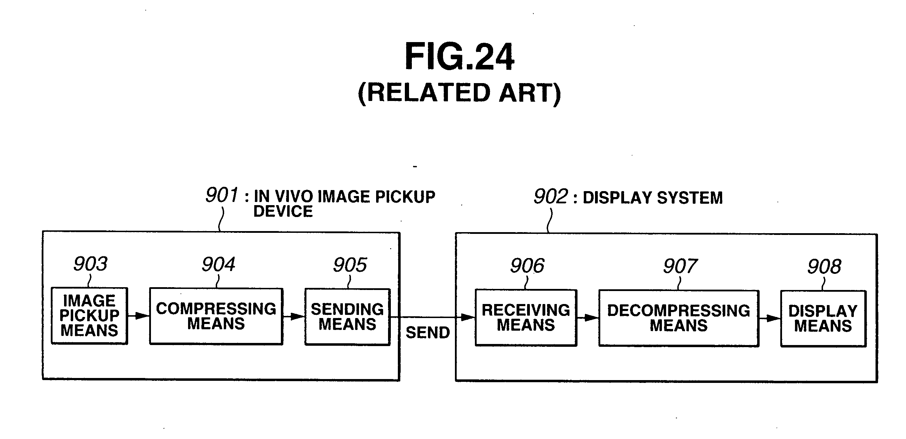

Fig. 24 is a block diagram showing the schematic structures of an in vivo image pickup device and a display system for displaying an image signal picked-up by the in vivo image pickup device, as disclosed in PCT WO03/010967.

Referring to Fig. 24, an in vivo image pickup device 901 comprises: image pickup means 903; compressing means 904; and sending means 905. The image pickup means 903 comprises a solid-state image pickup element that receives light from a subject and outputs a color image pickup signal in accordance with the amount of received light. The color image pickup signal outputted by the solid-state image pickup element is inputted to the compressing means 904 as an image signal. The compressing means 904 compresses the image signal and generates code data so as to effectively transfer the image signal. The sending means 905 sends the code data outputted by the compressing means 904 to an extracorporeal display system 902 by wireless communication.

The display system 902 comprises: receiving means 906; decompressing means 907, and display means 908. The receiving means 906 comprises an antenna for reception, and receives the code data sent in vivo via the antenna. The decompressing means 907 decompresses the code data received by the receiving means 906 and generates the image signal. The display means 908 displays the image signal generated by the decompressing means 907, and the displayed image signal is used for diagnosis.

However, PCT WO03/010967 does not describe the in vivo image pickup device having means for correcting the defective pixel. The image pickup element in the in vivo image pickup device contains a defective pixel, in particular, compression is performed by using the correlativity with a neighborhood pixel, the compression thus applies harmful affection to a normal pixel from the defective pixel. In such a case, there is a danger that the advantage for correction is not sufficiently obtained after decompression in a display system because a defective portion is diffused in another pixel.

SUMMARY OF THE INVENTION

It is an object of the present invention to provide an in vivo image pickup device and an in vivo image pickup system that preferably obtain an image signal by a correcting function of a defective pixel before compressing an image signal in the in vivo image pickup device.

According to the present invention, an in vivo image pickup device that is inserted in the body cavity and generates and sends an image signal of a subject, the in vivo image pickup device comprises: an image pickup unit that has a plurality of pixels with arrays on a light receiving surface thereof and converts a subject image formed on the light receiving surface into the image signal; a defect correcting circuit that corrects the image signal of a defective pixel of the image pickup unit; a compressing circuit that compresses the image signal from the defect correcting circuit; and a sending circuit that sends the compressed image signal.

With the above-mentioned structure, a defect correcting circuit corrects an image signal of a defective pixel of an image pickup unit and a compressing circuit then compresses the image signal outputted from the image pickup unit. A sending circuit sends the compressed image signal. Therefore, the defective pixel is corrected before the compressing circuit compresses the image signal, and the sending circuit sends the image signal out of the body cavity.

Preferably, the defect correcting circuit comprises a detecting circuit for detecting the defective pixel and a correcting circuit for correcting the image signal of the detected defective pixel.

Further, with the structure, the detecting circuit included in the defect correcting circuit detects the defective pixel of the image signals outputted from the image pickup unit and the correcting circuit then corrects the image signal of the detected defective pixel. Therefore, the defect correcting circuit detects the defective pixel and corrects the defective pixel.

Preferably, the defect correcting circuit further comprises a storing circuit for storing the position of the defective position.

Furthermore, with the structure, the storing circuit included in the defect correcting circuit stores the position of the defective pixel. The storing circuit stores the position of the defective pixel and therefore the position of the defective pixel is used for correction.

BRIEF DESCRIPTION OF THE DRAWINGS

Fig. 1 is a diagram showing the structure of an in vivo image pickup system according to first to fifth embodiments of the present invention;

Fig. 2 is a diagram showing the structure of an in vivo image pickup device according to the first embodiment of the present invention;

Fig. 3 is a diagram showing one positional relationship of a pixel for determining a defect according to the first embodiment of the present invention;

Fig. 4 is a diagram showing another positional relationship of the pixel for determining the defect according to the first embodiment of the present invention;

Fig. 5 is a diagram for explaining a determining method of a defective pixel according to the first embodiment of the present invention;

Fig. 6 is a diagram for explaining a correcting method of the defective pixel according to the first embodiment of the present invention;

Fig. 7 is a diagram showing the structure of a defect correcting circuit in the in vivo image pickup device according to the second embodiment of the present invention;

Fig. 8 is a diagram showing one positional relationship of the pixel for determining the defect according to the second embodiment of the present invention;

Fig. 9 is a diagram showing another positional relationship of the pixel for determining the defect according to the second embodiment of the present invention;

Fig. 10 is a diagram showing another positional relationship of the pixel for determining the defect according to the second embodiment of the present invention;

Fig. 11 is a diagram for explaining a determining method of the defective pixel according to the second embodiment of the present invention;

Fig. 12 is a diagram showing an example of linear interpolation between two points according to the second embodiment of the present invention;

Fig. 13 is a diagram showing the structure of an in vivo image pickup device according to the third embodiment of the present invention;

Fig. 14 is a diagram showing one positional relationship of the pixel for determining the defect according to the third embodiment of the present invention;

Fig. 15 is a diagram showing another positional relationship of the pixel for determining the defect according to the third embodiment of the present invention;

Fig. 16 is a diagram showing another positional relationship of the pixel for determining the defect according to the third embodiment of the present invention;

Fig. 17 is a diagram showing an example of linear interpolation at 8 neighborhood pixels according to the third embodiment of the present invention;

Fig. 18 is a diagram for explaining the operation for creating a luminance signal Y and color difference signals Cb and Cr of color signals R, G, and B in a pre-processing circuit according to the third embodiment of the present invention;

Fig. 19 is a diagram showing the structure of a defect correcting circuit and a compressing circuit in an in vivo image pickup device according to the fourth embodiment of the present invention;

Fig. 20 is a diagram for explaining a determining method of the defective pixel according to the fourth embodiment of the present invention;

Fig. 21 is a diagram for explaining a correcting method of the defective pixel according to the fourth embodiment of the present invention;

Fig. 22 is a diagram showing frames of the color signals R, G, and B according to the fourth embodiment of the present invention;

Fig. 23 is a diagram showing the structure of an image pickup unit in an in vivo image pickup device according to the fifth embodiment of the present invention; and

Fig. 24 is a block diagram showing the schematic structure of an in vivo image pickup device and the schematic structure of a display system for displaying an image signal picked-up by the in vivo image pickup device according to a conventional art.

DESCRIPTION OF THE PREFERRED EMBODIMENTS

Embodiments of the present invention will be described with reference to the drawings.

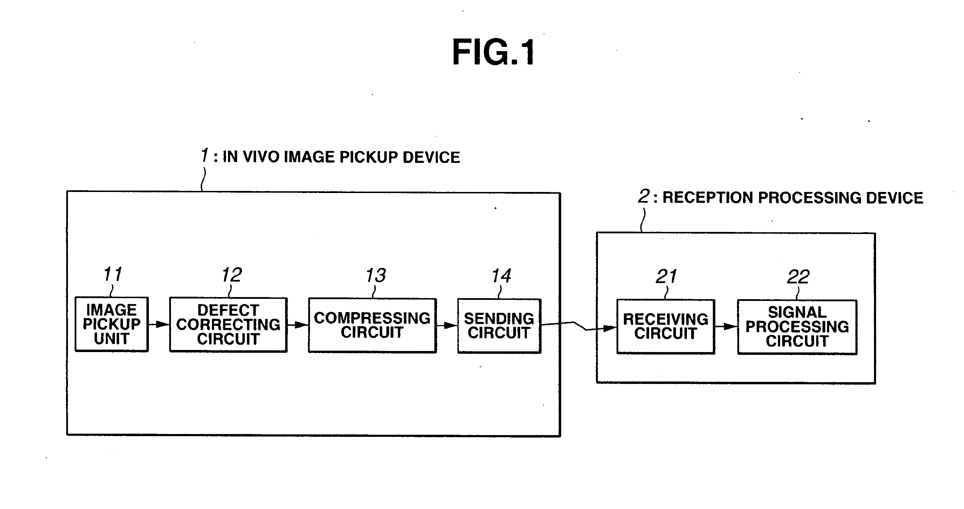

Fig. 1 is a diagram showing the structure of an in vivo image pickup system according to first to fifth embodiments of the present invention.

Referring to Fig. 1, the in vivo image pickup system comprises: an in vivo image pickup device 1 that is inserted in the body cavity and generates and sends an image signal of a subject; and a reception processing device 2 that is arranged separately from the in vivo image pickup device 1 inserted in the body cavity and receives and processes the image signal sent from the in vivo image pickup device 1.

The in vivo image pickup device 1 comprises: an image pickup unit 11 that has a plurality of pixels on the light receiving surface thereof and converts a subject image formed on the light receiving surface into the image signal; a defect correcting circuit 12 that corrects the image signal of the defect pixel of the image pickup unit 11; a compressing circuit 13 that compresses the image signal from the defect correcting circuit 12; and a sending circuit 14 that sends the compressed image signal. Since the in vivo image pickup device 1 is inserted in the body cavity, a battery or a system for extracorporeally supplying power by wireless communication may be used as a power supply of the in vivo image pickup device 1.

The reception processing device 2 comprises a receiving circuit 21 that receives the image signal sent from the sending circuit 14; and a signal processing circuit 22 that performs predetermined signal processing of the image signal received by the receiving circuit 21.

A description is given of the entire operation of the common system according to the first to fifth embodiments of the present invention with reference to Fig. 1.

In the in vivo image pickup device 1, first, the image signal picked-up by the image pickup unit 11 is inputted to the defect correcting circuit 12. The defect correcting circuit 12 processes the picked-up image signal by a predetermined defect correcting method, and then outputs the processed signal to the compressing circuit 13. The compressing circuit 13 generates a luminance signal Y and color difference signals Cb and Cr, and compresses the image signal using a predetermined compressing method, such as JPEG or MPEG. The sending circuit 14 sends the image signal compressed by the compressing circuit 13 to the reception processing device 2 outside the body cavity by wireless communication. With a predetermined compressing method, such as JPEG or MPEG, the compression is performed by using the correlativity with a neighborhood pixel. With the above structure, the defect is corrected before compression, thereby preventing the danger of harmful affection to a normal pixel from the defective pixel.

In the reception processing device 2, the receiving circuit 21 receives the image signal sent from the in vivo image pickup device 1 and the signal processing circuit 22 performs predetermined signal processing of the image signal received by the receiving circuit 21.

(First embodiment)

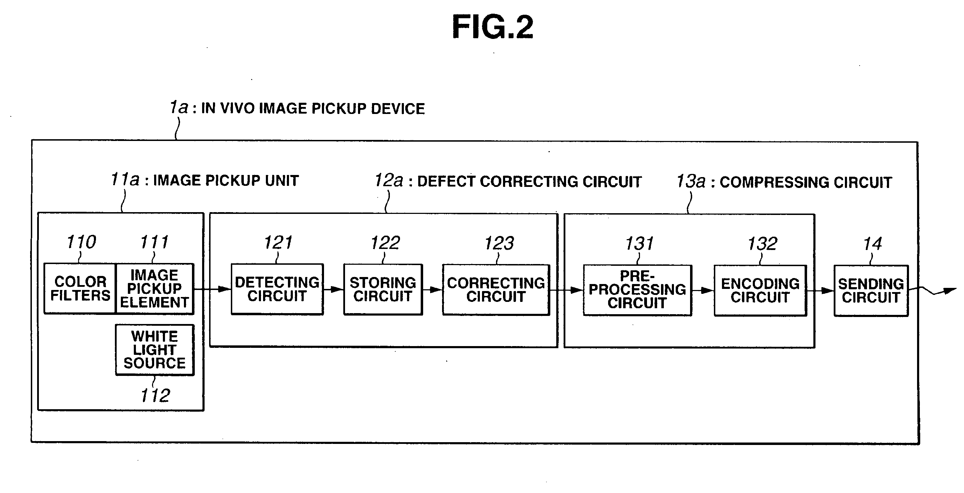

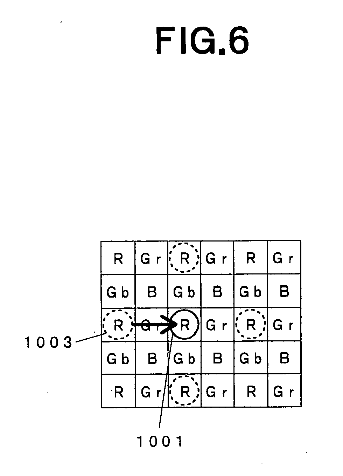

Next, the first embodiment of the present invention will be described in accordance with Figs. 2 to 6.

Fig. 2 is a diagram showing the structure of an in vivo image pickup device according to the first embodiment of the present invention.

Referring to Fig. 2, reference numeral 1a denotes an in vivo image pickup device that is inserted in the body cavity, and generates and sends an image signal of a subject. The in vivo image pickup device 1a comprises: an image pickup unit 11a; a defect correcting circuit 12a; a compressing circuit 13a; and the sending circuit 14.

The image pickup unit 11a comprises: an image pickup element 111, such as a CMOS, to which color filters 110 are adhered with an RGB bayer array; and a white light source 112, such as an LED, that illuminates in vivo.

The defect correcting circuit 12a is a defect correcting circuit that corrects the image signal of the defective pixel of the image pickup element 111, and comprises: a detecting circuit 121 that detects a defective pixel of the image pickup element 111 based on the comparison of the maximum and minimum levels of the same four neighborhood pixels, upon turning on the power; a storing circuit 122 that stores the coordinates of the defective pixel; and a correcting circuit 123 that replaces the defective pixel with a value of a just-before pixel having the same color.

The compressing circuit 13a is a compressing circuit that compresses the image signal from the defect correcting circuit 12a, and further comprises: a pre-processing circuit 131 that inputs the image signal from the defect correcting circuit 12a, and generates the luminance signal Y and color difference signals Cb and Cr; and an encoding circuit 132 that compresses the image signal by using a predetermined compressing method, such as JPEG or MPEG.

Next, a description is given of the operation of the in vivo image pickup device 1a according to the first embodiment of the present invention with reference to Fig. 2.

The in vivo image pickup device 1a according to the first embodiment detects the defective pixel of the image pickup element 111, in the start of device upon truing on the power. For example, the power is turned on while the in vivo image pickup device 1a is packed, then, the LED emits light, the front wall in the pack having a white inner wall is shot, and a white-image signal on the entire surface is captured. After that, the image signal is captured while the LED is turned off. Since the inside of pack is shielded, the image thereof forms a black image signal on the entire surface.

Hereinbelow, a description is given of an example of operation of the detecting circuit 121 in the defect correcting circuit 12a, for detecting the defective position of pixel based on the image signals of two images individually having the entire white and black colors, when digital data contains 8 bits, serving as input data of pixels.

The image pickup element 111 according to the first embodiment has color filters 110 with RGB bayer array adhered thereto. In the white image signals on the entire image, when a pixel value of a target pixel is 180 and pixel values of four neighborhood pixels having the same color are 230, 235, 232, and 240, the minimum value is 230 and the target pixel is smaller than the minimum value by 50. Here, an allowable smaller value from the minimum value, that is, threshold in the smaller direction is considered.

In this case, when a threshold for determining the defect of the target pixel is set as 60, the pixel value of 50 is within the threshold of 60. Thus, the target pixel is determined as a normal pixel. If the threshold is set as 40, the pixel value of 50 is over the threshold of 40. Thus, the target pixel is determined as a defective pixel. When the target pixel is a defective one, the coordinates of the target pixel are stored in the storing circuit 122 in the defect correcting circuit 12a. Thus, the defective pixel having a black color is detected.

Similarly, in the black image signals on the entire image, when a maximum value of four pixels having the same color near the target pixel is 20, a target-pixel value is 70, and a threshold is set as 40, the detecting circuit 121 in the defect correcting circuit 12a determines that the target pixel larger than the maximum value by 50 is a defective pixel. The coordinates of the target pixel is additionally stored in the storing circuit 122 in the defect correcting circuit 12a.

Thus, the defective pixel of the white image signal is detected. After the above detection, the in vivo image pickup device 1a is extracted from a pack, is swallowed, and is captured in the body. The image pickup unit 11a irradiates in vivo by the white light source 112, and the image pickup element 111 picks-up a color image signal of the living body.

The picked-up color image signal is processed in accordance with coordinate positional information of the defective pixel stored in the storing circuit 122. The defective pixel is corrected by the defect correcting circuit 12a, which will be described later. In the compressing circuit 13a, the pre-processing circuit 131 generates the luminance signal Y and the color difference signals Cb and Cr. Then, the encoding circuit 132 encodes the image signal to that of JPEG or MPEG, and the sending circuit 14 extracorporeally sends the encoded signal by wireless communication.

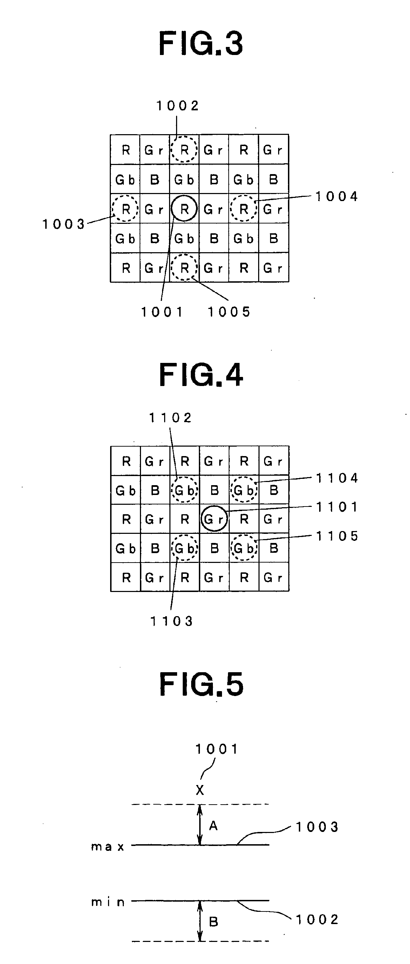

Figs. 3 and 4 are diagrams showing positional relationships of the pixel for determining the defect.

Referring to Figs. 3 and 4, color filters having RGB bayer array are adhered to the image pickup element. The same green (G) filter is used for the arrays of R and G of the first, third, ... columns and the arrays of G and B of the second, fourth, .... However, the G filter of the first, third, ... is referred to as a Gb filter and the G filter of the second, fourth, ... is referred to as a Gr filter so as to distinguish the G filter of the first, third, ... and the G filter of the second, fourth, ....

Referring to Fig. 3, in the determination as whether or not a target pixel 1001 of the filter R is a defective one, it is determined by using pixels 1002 to 1005 which have the same color and are located in up, down, right, and left of the target pixel 1001. The above-mentioned operation may be executed for the pixel of the filter B.

Referring to Fig. 4, in the determination as whether or not a target pixel 1101 of the filter Gr is a defective one, it is determined, by using pixels 1102 to 1105 in the diagonal direction of the filter Gb, whether or not the target pixel 1101 is a defective one. The above-mentioned operation may be executed by using the pixel in the diagonal direction of the filter Gr in the determination as whether or not the pixel of the filter Gb is a defective one.

In the natural image, the correlativity is higher in the up, down, right, and left pixels, instead of the pixel in the diagonal direction. Advantageously, the pixels in the up, down, right, and left direction are thus interpolated. However, the in vivo image has no directivity and therefore the correlativity is higher with the more neighborhood pixel. Thus, in the in vivo image pickup device, the defective pixel is detected and corrected with high precision.

According to the first embodiment, a description is given of determining whether or not the pixel is a defective one in the start operation of the in vivo image pickup device 1a. Or, it may be determined whether or not the pixel is a defective one during the operation of the in vivo image pickup device 1a. Hereinbelow, a method for determining whether or not the pixel will be described.

For example, it is determined, by using the pixels 1002 to 1005 having the same color in the up, down, right, and left directions, whether or not the target pixel 1001 shown in Fig. 3 is a defective one.

According to the first embodiment, the output of each pixel from the image pickup element 111 contains 8 bits, as digital data, and has values ranging 255 to 0.

Referring to Fig. 3, the maximum value of the pixels in the up, down, right, and left directions is 160 of the pixel 1003, the minimum value is 140 of the pixel 1002, a first threshold A is 80, and a second threshold B is 50. When the value of the target pixel 1001 is 245, Fig. 5 shows a relationship among a maximum value max, a minimum value min, the first threshold A, the second threshold B, and a value C of the target pixel 1001.

When the following conditional relation is satisfied, it is determined that the target pixel is a defective one.

(Formula 1-1)

Value of target pixel > maximum value + first threshold A, or

Minimum value - second threshold B > value of target pixel.

Based on (Formula 1-1), the value of target pixel 1001 has a relation of [245 > (160 + 80)]. Thus, the target pixel 1001 is detected as a defective pixel.

The maximum value and the minimum value of the pixel having the same color near the target pixel, and the two thresholds are used upon determining (detecting), during operation of the in vivo image pickup device 1a, whether or not the target pixel is a defective one.

Next, a correcting method of the defective pixel will be described.

According to the first embodiment, the target pixel 1001 is corrected by replacing the defective pixel with a value of the just-before pixel 1003 having the same color on the same line. With the above method, advantageously, the defective pixel can be corrected simple and fast and the contrast of the image signal is kept.

According to the first embodiment, as mentioned above, the detecting circuit 121 detects (determines) the defective pixel, the correcting circuit 123 corrects the defective pixel, and the image signal is preferably obtained. Further, since the defective pixel is corrected before compression, the danger of harmful influence to the normal pixel from the defective pixel is prevented. Further, it is possible to detect not only the initial defective pixel caused by the CCD material just before the manufacturing and the manufacturing process but also the subsequent defective pixel caused by the external environment of radiation and electrostatic destruction and the aging change.

Further, since the defective pixel is simply with high precision, the circuit scale is reduced, the long-time driving is possible with a battery having limited power capacity, and the power consumption is low.

(Second embodiment)

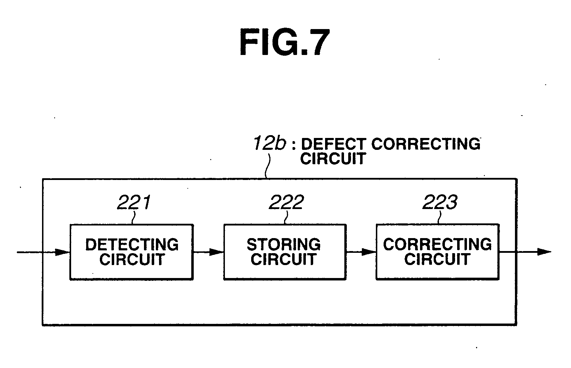

The second embodiment of the present invention will be described with reference to Figs. 7 to 12.

According to the second embodiment, a defect detecting method and a defect correcting method are different from those according to the first embodiment.

Fig. 7 is a diagram showing the structure of a defect correcting circuit 12b in the in vivo image pickup device according to the second embodiment of the present invention. The structures of the image pickup unit and the compressing circuit are the same as those shown in Fig. 2.

The defect correcting circuit 12b is a defect correcting circuit that corrects the image signal of the defective pixel of the image pickup element 111, such as a CCD, and comprises: a detecting circuit 221 that detects the defective pixel of the image pickup element 111 by the comparison with the average of values of two neighborhood pixels or of four neighborhood pixels having the same color for a predetermined period; a storing circuit 222 that stores the coordinate of the defective pixel; and a correcting circuit 223 that corrects the defective pixel by the linear interpolation (average) of the two neighborhood pixels or four neighborhood pixels.

Next, a description is given of the operation of the defect correcting circuit 12b according to the second embodiment of the present invention with reference to Fig. 7.

The detecting circuit 221 detects the defective pixel every predetermined period (time or number of captured images) by a detecting method of the defective pixel, which will be described later. A light source, such as an LED, is lit-off every predetermined period, the image is captured, and the defect is detected, thereby effectively detecting a white defective pixel.

The coordinate positional information of the target pixel detected as a defective pixel is stored in the storing circuit 222. The correcting circuit 223 corrects the defective pixel by a correcting method of the defective pixel, which will be described later.

Thus, the power for detecting and correcting the defective pixel is reduced, and the subsequent defective pixel is detected and corrected.

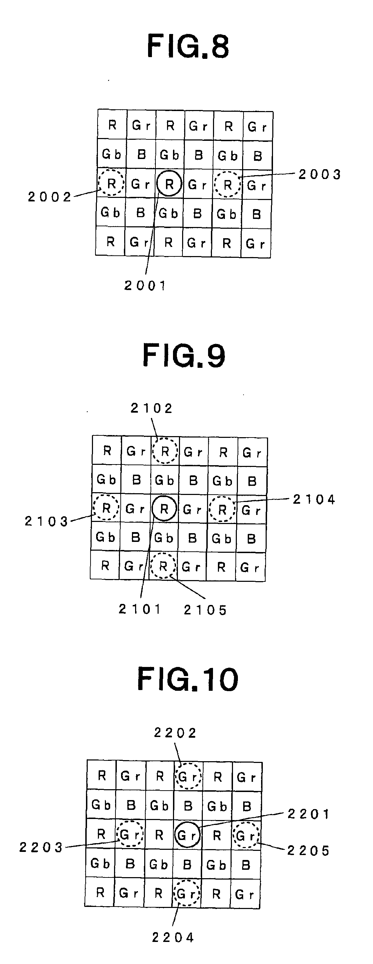

Figs. 8 to 10 are diagrams showing positional relationships of the pixel for determining the defect.

Referring to Fig. 8, a defect of an R pixel in a target pixel 2001 is determined by using two pixels 2002 and 2003 having the same color on the same line. The same operation is performed for B, Gr, and Gb pixels. In the determination of the defective pixel, referring to Fig. 9, it may be determined, by using four pixels 2102 to 2105 having the same color in the up, down, right, and left directions, whether or not the target pixel 2101 is a defective one. The same operation may be performed for the B pixel.

Referring to Fig. 10, the defect of the Gr or Gb pixel in the target pixel 2001 may be determined by using four pixels 2202 to 2205 having the same color in the up, down, right, and left directions. With the pixels having the same color in the up, down, right, and left direction, the defective pixel can be detected and interpolated between the Gr pixels and between the Gb pixels. In particular, in the case of a CCD having different transfer lines of image pickup charges of the Gr and Gb pixels, the defective pixel can be determined and interpolated with high precision. Incidentally, in the case of a CCD having the same transfer lines of image pickup charges of the Gr and Gb pixels, similarly to that described with reference to Fig. 4 according to the first embodiment, the defective pixel of the Gr pixel is determined by using the Gb pixel in the diagonal direction, and the defective pixel of the Gb pixel is determined by using the Gr pixel in the diagonal direction.

Next, a description is given of the determining method of the defective pixel with reference to Fig. 10.

In the example shown in Fig. 10, it is determined, by using the neighborhood pixels 2202 to 2205 having the same color in the up, down, right, and left directions, whether or not a target pixel 2201 is a defective pixel.

According to the second embodiment, the output of each pixel from the image pickup element 111 contains 8 bits, as digital data, and has values ranging 255 to 0.

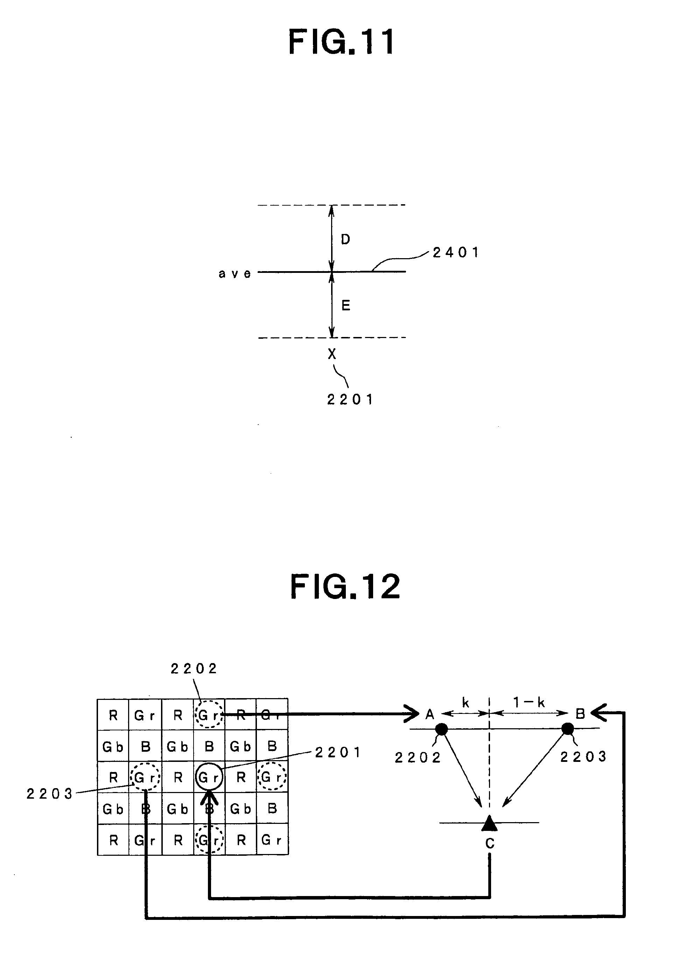

Referring to Fig. 10, an average pixel value ave of pixels 2202 to 2205 is 180, a third threshold D is 30, and a fourth threshold E is 60. When the value of a target pixel 2201 is 100, a relationship is obtained as shown in Fig. 11. Reference numeral 2401 denotes an average of the four pixels 2202 to 2205 in the up, down, right, and left directions.

When the following conditional relation is satisfied, it is determined that the target pixel is a defective one.

```
(Formula 1-2)

Value of target pixel > average of neighborhood pixels

+ third threshold D, or

average of neighborhood pixels - fourth threshold E >

value of target pixel.
```

Based on (Formula 1-2), the value of target pixel 2201 has a relation of 100 < (180 - 60). Thus, the target pixel 2201 is detected as a defective pixel.

The defective pixel is determined by using the average of neighborhood pixels having the same color and the two thresholds as mentioned above.

Here, values of the third threshold D and the fourth threshold E may be changed in accordance with the average signal level of neighborhood pixels. When the average signal level of neighborhood pixels having the same color is low, the value of the third threshold D is high and the value of the fourth threshold E is low. On the other hand, when the average signal level of neighborhood pixels having the same color is high, the value of the third threshold D is low and the value of the fourth threshold E is high.

The four neighborhood pixels 2202 to 2205 having the same color in the up, down, right, and left directions shown in Fig. 10 have values of 230, 160, 200, and 30, respectively. Then, the defective pixel may be determined from the pixels excluding the pixel having the maximum signal level and the pixel having the minimum signal level. In this case, the pixels 2202 and 2205 are excluded.

Thus, if the pixels in the up, down, right, and left directions include the defective pixel that is not detected, the affection from the defective pixel is prevented. Advantageously, the variation in signal level is suppressed and the defective pixel is detected with high precision.

When the four neighborhood pixels having the same color in the up, down, right, and left directions shown in Fig. 10 include the defective pixel that has already been detected, the defective pixel may be determined from the pixels from which the defective pixel that has already been detected is excluded. With the methods, in the case of continuous defective pixels, the defective pixel is detected.

In the determination of the defective pixel, not only the pixels having the same color in the up, down, right, and left directions but also the neighborhood pixel having another color may be used. When the defective pixel is determined by the pixels having the same color in the up, down, right, and left directions, the erroneous detection of the defective pixel can be prevented with high precision by determining again, based on information on an absolute or degree of variation in neighborhood pixels having another color and an inclination of the value, whether or nor the target pixel is a defective one.

Next, a description is given of the correcting method of the detected defective pixel with reference to Fig. 12.

The linear interpolation is used for the pixel values of neighborhood pixels in the up, down, right, and left directions, a corrected pixel value is generated, and the pixel value of the target pixel is replaced with the corrected pixel value, thereby correcting the defective pixel.

Fig. 12 shows an example of linear interpolation between two points. Referring to Fig. 12, pixel values of normal pixels 2202 and 2203 are designated by reference numerals A and B, respectively, and the corrected pixel value of the target pixel is designated by reference numeral C. Then, the pixel value C is obtained based on the linear interpolation equation of (Formula 1-3).

$$(\text{Formula } 1\text{-}3)$$

$$A * (1 - k) + B * k = C$$

Here, reference numeral k denotes a coefficient corresponding to the distance between the pixels 2202 and 2203 in Fig. 12 corresponding to the pixel values A and B for the target pixel, and is a real number ($0 \le k \le 1$). In the example shown in Fig. 12, k is equal to 0.5. Reference symbol * denotes the multiplication.

The value of the target pixel 2201 is replaced with the pixel value C obtained by the linear interpolation between the pixel values A and B of the normal pixels, serving as the corrected pixel values. With the method, advantageously, a relatively smooth image signal is obtained, and the image signal can be processed relatively fast. Since the pixels of the image pickup element are generally arranged at an identical interval, any specific circuit for linear interpolation is not necessary, the target pixel may be replaced with the average ave (refer to Fig. 11) of the neighborhood pixels having the same color used for determination of the pixel defect.

As mentioned above, according to the second embodiment, the same advantages as those according to the first embodiment are obtained. That is, the detecting circuit 221 detects (determines) the defective pixel, the correcting circuit 223 corrects the defective pixel, and the image signal is preferably obtained. The defective pixel is corrected before compression. Therefore, the danger of the harmful affection to the normal pixel from the defective pixel is prevented. Further, according to the second embodiment, the defective pixel is detected and corrected with higher precision, as compared with the first embodiment. Furthermore, it is possible to detect not only the initial defective pixel caused by a CCD material just before the manufacturing and the manufacturing process but also the subsequent defective pixel caused by the external environment of radiation and electrostatic destruction and the aging change.

Further, since the defective pixel is simply with high precision, the circuit scale is reduced, the long-time driving is possible with a battery having limited power capacity, and the power consumption is low.

(Third embodiment)

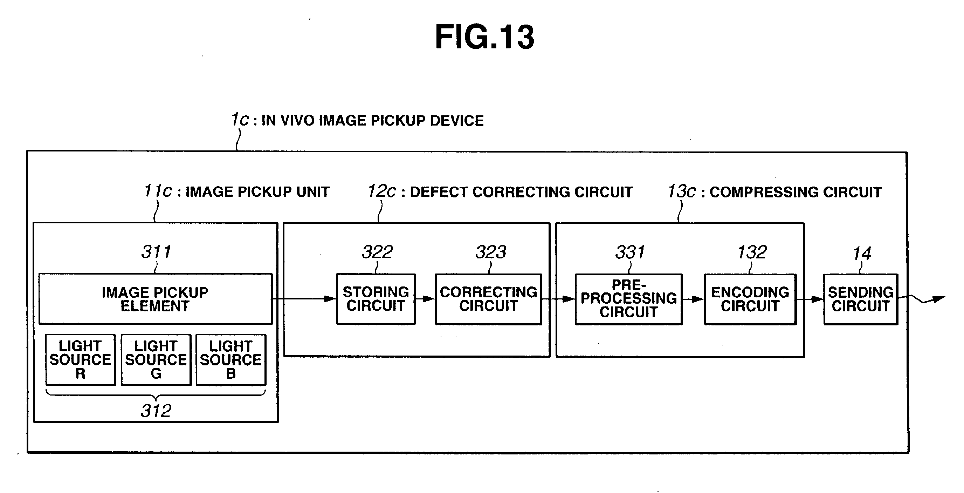

The third embodiment of the present invention will be described with reference to Figs. 13 to 18.

Fig. 13 is a diagram showing the structure of an in vivo image pickup device according to the third embodiment of the present invention.

Referring to Fig. 13, reference numeral 1c is an in vivo image pickup device that is inserted in the body cavity, and generates and sends an image signal of a subject. The in vivo image pickup device 1c comprises: an image pickup unit 11c; a defect correcting circuit 12c; a compressing circuit 13c; and the sending circuit 14.

The image pickup unit 11c comprises: an image pickup element 311, such as an NMOS, to which a color filter is not adhered; and a light source 312, serving as a light emitting element, that emits red (R), green (G), and blue (B) and is controlled to sequentially emit one color of light every frame.

The defect correcting circuit 12c is a defect correcting circuit that corrects the image signal of the defective pixel of the image pickup element 311, and comprises: a storing circuit 322 that records the coordinates of the defective pixel of the image pickup element 311 from the shipping timing; and a correcting circuit 323 that corrects the defective pixel by the linear interpolation of the neighborhood pixels having the same color. Since the storing circuit 322 records, in advance, the coordinates of the defective pixel at the shipping timing, the detecting circuit of the defective pixel shown in Fig. 2 according to the first embodiment is deleted.

The compressing circuit 13c is a compressing circuit that compresses the image signal from the defect correcting circuit 12c, and comprises: a pre-processing circuit 331 that generates the luminance signal Y and the color difference signals Cb and Cr based on the continuous image data of R, G, and B; and the encoding circuit 132 that encodes an image signal from the pre-processing circuit 331 to image data of JPEG or MPEG.

According to the third embodiment, the pre-processing circuit 331 functions as combining means that combines one color image signal based on the image data of three continuous frames R, G, and B. The encoding circuit 132 functions as compressing circuit that compresses the output of the image signal from the combining means.

The compressed image signal is externally sent to the sending circuit 14 by wireless communication.

The compressing circuit may comprise only the encoding circuit, and the pre-processing circuit may be arranged separately from the compressing circuit.

Next, a description is given of the operation of the in vivo image pickup device 1c according to the third embodiment of the present invention with reference to Fig. 13.

The in vivo image pickup device 1c is swallowed to be captured in the body. When the in vivo image pickup device 1c reaches the image pickup position, the in vivo image pickup device 1c picks-up the image. In the image pickup unit 11c, the light source 312 sequentially emits red, green, and blue LEDs one by one every frame, and the image pickup element 311 picks-up the image signals of the three frames.

The storing circuit 322 stores, in advance, the defect positional information of the image pickup element 311 at the shipping timing. The correcting circuit 323 corrects the defective pixels of the image signals of the three frames picked-up by the image pickup element 311 based on the defect positional information, with a correcting method, which will be described later.

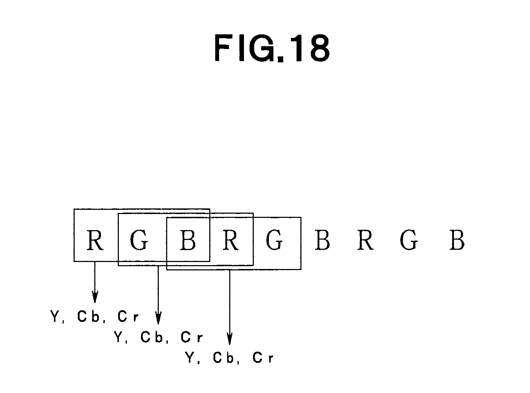

The image signal is inputted to the compressing circuit 13c. Referring to Fig. 18, the pre-processing circuit 331 in the compressing circuit 13c generates the luminance signal Y and the color difference signals Cb and Cr every frame from the continuous R, G, B/G, B, R/B, R, G/... surrounded by square frames based on sequential light R, G, B, R, G, B, ... of light R, G, and B sequentially emitted every frame. The encoding circuit 132 encodes the image signals to image data of JPEG or MPEG. The sending circuit 14 extracorporeally sends the data.

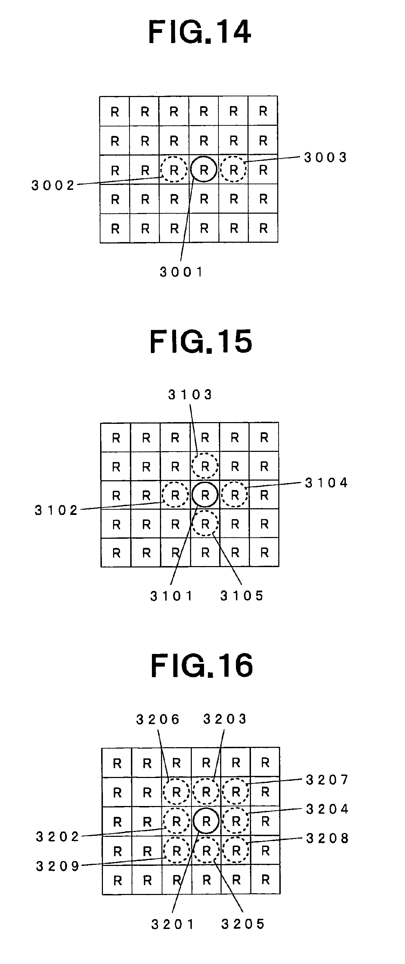

Figs. 14 to 16 are diagrams showing positional relationships of defective pixels that are corrected.

Since the light source 312, serving as the light emitting elements R, G, and B, is controlled to sequentially emit light of one color every frame, the defective pixel of the one-color image signal is corrected. Referring to Figs. 14 to 16, a red image signal is used as an example, and a green image and a blue image signal have the same pixel positional relationship as that of the red image signal.

Fig. 14 shows an example of correcting a defect of a defective pixel 3001 by using pixels 3002 and 3003 on the same line. Fig. 15 shows an example of correcting a defect of a defective pixel 3101 by using pixels 3102 to 3105 in the up, down, right, and left direction. Fig. 16 shows an example of correcting a defect of a defective pixel 3201 by using pixels 3202 to 3209 having the pixels in the diagonal direction in addition to the pixels in the up, down, right, and left directions.

Next, the defect correcting method will be described.

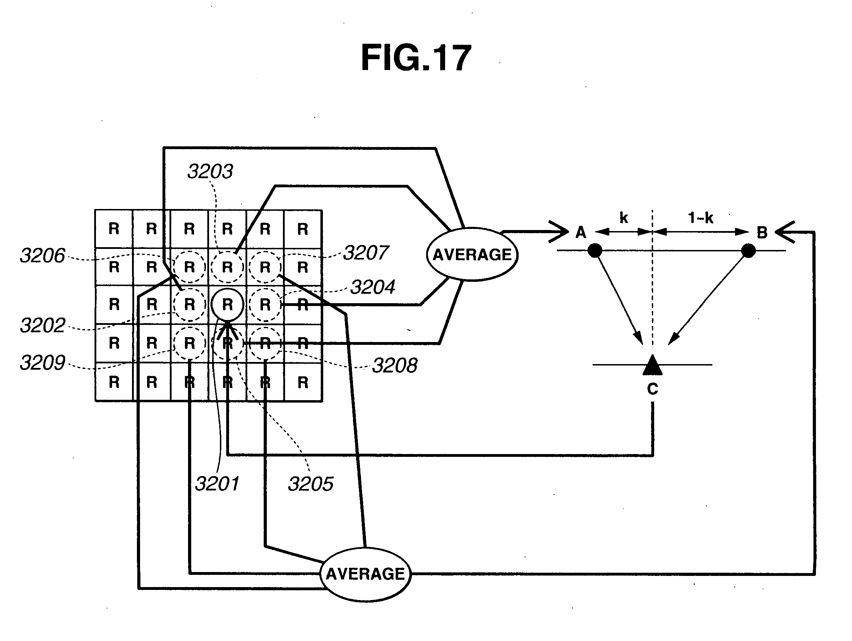

The defect is corrected by replacing the defective pixel by using the linear interpolation (weighted average value) of pixels in the up, down, right, and left directions and in the diagonal direction, serving as normal pixels shown in Fig. 16. Fig. 17 shows an example of the linear interpolation of eight neighborhood pixels.

Referring to Fig. 17, reference numeral A denotes an average of four pixels 3202 to 3205 in the horizontal and vertical directions, and reference numeral B denotes an average of four pixels 3206 to 3209 in the diagonal direction. A corrected value C is obtained, serving as a weighted average in inverse proportional to the distance, based on the averages A and B by using a linear interpolation formula (Formula 1-3).

$$(\text{Formula 1-3})$$

$$A*(1-k) + B*k = C,$$

where k is a coefficient corresponding to the distance to the pixels used in the case of obtaining the averages A and B with respect to the defective pixel, and is a real number (0 ≤ k ≤ 1). In the example shown in Fig. 17, k is 0.4.

According to the third embodiment, as mentioned above, advantageously, a relatively smooth image signal is obtained and the signal is relatively fast processed. When the defective pixel exists near the target pixel, the defective pixel is corrected with higher precision by using the average of the pixels excluding the defective pixel.

(Fourth embodiment)

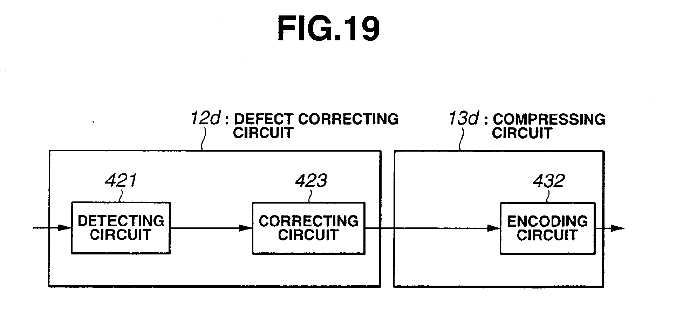

Next, the fourth embodiment of the present invention will be described with reference to Figs. 19 to 22.

The fourth embodiment is different from the third embodiment in the defect detecting method, the defect correcting method, and the pre-processing method of compression.

Fig. 19 is a diagram showing the structure of a defect correcting circuit and a compressing circuit in an in vivo image pickup device according to the fourth embodiment of the present invention. Further, Fig. 19 shows only the structures of a defect correcting circuit 12d and a compressing circuit 13d, serving as different structures from those shown in Fig. 13 according to the third embodiment, omitting the image pickup unit and the sending circuit similar to those shown in Fig. 13.

Referring to Fig. 19, the reference numeral 12d denotes a defect correcting circuit that corrects the image signal of the defective pixel of the image pickup element 311, and comprises: a detecting circuit 421 that detects the defective pixel of the image pickup element 311 from a near pixel value on the same line; and a correcting circuit 423 that corrects the defective pixel from the near pixel value on the same line. The defect correcting circuit 12d detects and corrects the defective pixel for each image pickup operation. Therefore, a storing circuit that stores the coordinate of the position of the defective pixel as shown in Fig. 13 is deleted.

The reference numeral 13d denotes a compressing circuit that compresses the image signal from the defect correcting circuit 12d, and comprises only an encoding circuit 432 that encodes the image signal to data of JPEG or MPEG.

Next, a description is given of the operation of the defect detecting circuit 12d and the compressing circuit 13d according to the fourth embodiment of the present invention.

The image signals of three R, G, and B frames picked-up by the image pickup unit 11c similar to that according to the third embodiment are inputted to the defect correcting circuit 12d. The detecting circuit 421 detects the defect by a defect detecting method, as will be described later. Subsequently, the correcting circuit 423 corrects the defect by a correcting method, which will be described later. According to the fourth embodiment, the defective pixel is detected and corrected for each image pickup operation and therefore the defect is appropriately corrected in accordance with the image pickup signal. It is possible to detect not only the initial defective pixel but also the subsequent defective pixel.

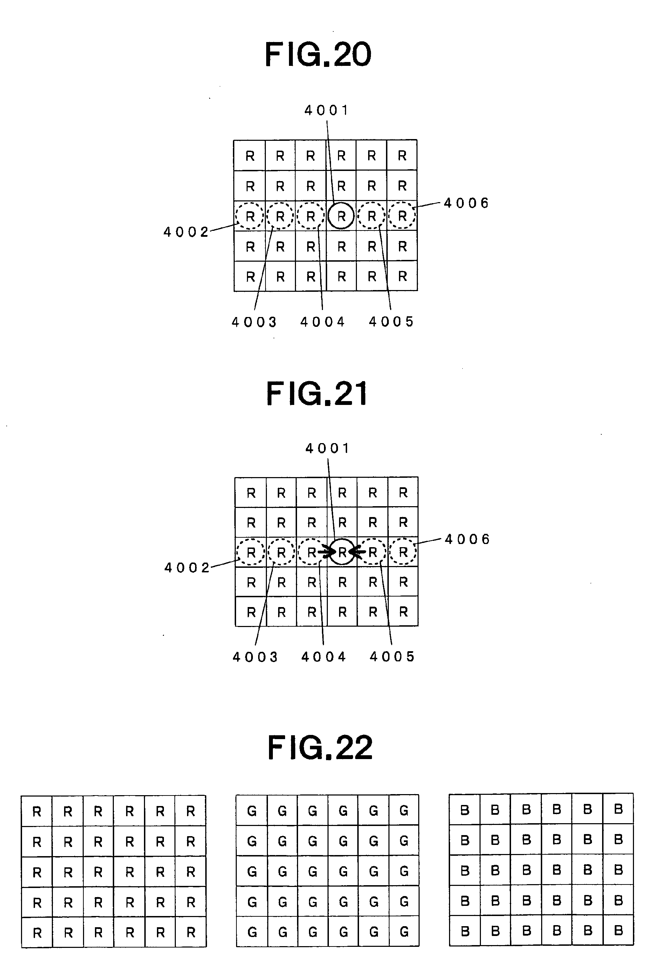

The image signal is inputted to the compressing circuit 13d. Referring to Fig. 22, the encoding circuit 432 individually encodes color information of R, G, and B, and the sending circuit 14 extracorporeally sends the encoded data.

According to the fourth embodiment, referring to Fig. 20, it is determined, by using neighborhood pixels 4004 and 4005 on the same line as that of a target pixel 4001, whether or not the target pixel 4001 is a defective pixel. In the determination of the defective pixel, similarly to the description with reference to Fig. 11, the average of pixels of the neighborhood pixels 4004 and 4005 is obtained, then, the target pixel 4001 is determined as a defective pixel when a value of the target pixel 4001 is the third threshold D or more from the average, or is the fourth threshold E or less from the average. Incidentally, it may be determined, by using pixels 4002 to 4006 in the front and back directions, whether or not the target pixel 4001 is a defective one. In the determination of the defective pixel with the pixels in the front and back directions, it may be determined, by using the maximum value and minimum value of values of neighborhood pixels mentioned above with reference to Fig. 5, whether or not the target pixel is a defective one, in addition to the determination of the defective pixel with the average shown in Fig. 11. The red (R) pixel is used in Fig. 20 and the green (G) and blue (B) pixels can be similarly used.

Next, the defect correcting method will be described.

When it is determined the target pixel 4001 is a defective one, the defect is corrected by replacing the target pixel 4001 with a pixel on the same line as that of the target pixel 4001. Referring to Fig. 21, the defective pixel may be corrected by the average of values of the neighborhood pixels 4004 and 4005, or a value of the target pixel 4001 may be determined by applying a multi-degree equation or weighted average of values of the pixels 4002 to 4006. According to the fourth embodiment, a defect is detected and is simultaneously corrected and a memory, serving as a storing circuit for storing the detecting position of the defective pixel, is not therefore necessary. Only the pixel on the same line is used for detection and correction of defective pixel and pixel information of the line on the front or back direction is not used, and a line memory is not therefore necessary. Data is corrected fast with a small-scaled circuit. The pixels having the same color continuously exist, the value of the defective pixel is replaced with an average of values of near pixels, the interpolated pixels are therefore continuous, and high advantages for correction are obtained.

As mentioned above, according to the fourth embodiment, the same advantages as those according to the third embodiment are obtained. That is, a relatively smooth image signal is obtained and, advantageously, data is fast processed. Further, according to the fourth embodiment, the image signals are encoded, for each R, G, and B frames, to data of JPEG or MPEG, and are outputted and sent. Therefore, a pre-processing circuit is not necessary.

(Fifth embodiment)

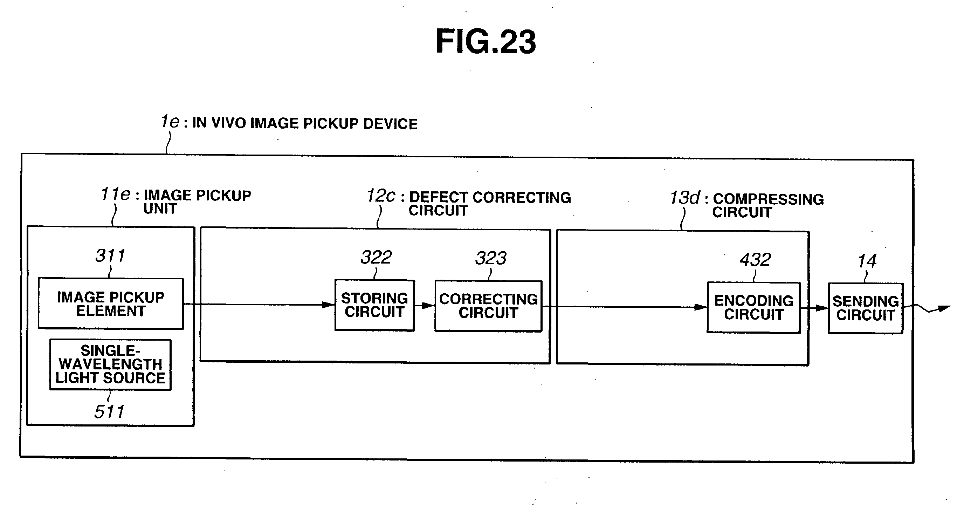

Next, the fifth embodiment of the present invention will be described. Fig. 23 shows the structure of an in vivo image pickup device according to the fifth embodiment.

An in vivo image pickup device le according to the fifth embodiment comprises: an image pickup unit lle according to the fifth embodiment; the defect correcting circuit 12c according to the third embodiment; and the compressing circuit 13d and the sending circuit 14 according to the fourth embodiment. Therefore, defect positional information of the image pickup element 311 is stored in the storing circuit 322 at the shipping timing in advance. The correcting circuit 323 corrects the defective pixel of the image signal picked-up by the image pickup element 311 based on the defect positional information.

The image signal is inputted to the compressing circuit 13d, the encoding circuit 432 encodes the encoded signal to data of JPEG or MPEG, and the sending circuit 14 extracorporeally sends the data.

Fig. 23 is a diagram showing the structure of the image pickup unit lle according to the fifth embodiment. The image pickup unit 11e comprises the image pickup element 311, such as a CCD, a CMOS, or an NMOS, with the sensitivity to a specific wavelength; and a light source 511 that emits light with a specific wavelength. The light source 511 is, e.g., a single-wavelength light source.

Next, a description is given of the operation of the image pickup unit 11e according to the fifth embodiment of the present invention with reference to Fig. 23.

Before examination, an examinee swallows, in advance, a medical drug that brightens a cancer cell in reaction to light with a specific wavelength. Then, the examinee swallows the in vivo image pickup device le. The in vivo image pickup device le reaches the desired image pickup position and the light source 511 thereafter emits light, and the image pickup element 311 picks-up the in vivo image. Since the medical drug in reaction to the light with a specific wavelength is swallowed in advance, the light source 511 emits light and the cancer cell is effectively found.

According to the fifth embodiment, as mentioned above, the same advantages as those according to the third and fourth embodiments are obtained. Further, according to the fifth embodiment, the cancer cell is effectively detected.

The present invention has been described according to the first to fifth embodiments. Further, it is possible to embody, by combining various methods, the light source and the image pickup element in the image pickup unit, the structures of the detecting circuit and the correcting circuit in the defect correcting circuit, and the structure of the pre-processing circuit of the compressing circuit according to the present invention.

According to the present invention, the defective pixel is corrected before compression and the danger of harmful affection to the normal pixel from the defective pixel is prevented. Further, the detecting circuit detects the defective pixel, the correcting circuit corrects the defective pixel, and the image signal is preferably obtained. Furthermore, the defective pixel is detected by using a plurality of neighborhood pixels, a corrected value of the defective pixel is further obtained by the plurality of neighborhood pixels used for detection, and a circuit for detecting and correcting the defective pixel is therefore simplified.

The present invention is not limited to the in vivo image pickup device and the in vivo image pickup system, and can be widely used for detecting and correcting the defective pixel in an image pickup device, such as a digital camera.

Having described the preferred embodiments of the invention referring to the accompanying drawings, it should be understood that the present invention is not limited to those precise embodiments and various changes and modifications thereof could be made by one skilled in the art without departing from the spirit or scope of the invention as defined in the appended claims.

## Claims

1. An in vivo image pickup device that is inserted in the body cavity and generates and sends an image signal of a subject, the in vivo image pickup device comprising:

   an image pickup unit that has a plurality of pixels with arrays on a light receiving surface thereof and converts a subject image formed on the light receiving surface into the image signal;
   a defect correcting circuit that corrects the image signal of a defective pixel of the image pickup unit;
   a compressing circuit that compresses the image signal from the defect correcting circuit; and
   a sending circuit that sends the compressed image signal.

2. An in vivo image pickup device according to Claim 1, wherein the defect correcting circuit comprises a detecting circuit that detects the defective pixel and a correcting circuit that corrects the image signal of the detected defective pixel.

3. An in vivo image pickup device according to Claim 2, wherein the defect correcting circuit further comprises a storing circuit that stores the position of the defective pixel.

4. An in vivo image pickup device according to Claim 1, wherein the image pickup unit comprises a CCD, a CMOS image pickup element, or an NMOS image pickup element, to which a color filter is adhered on the light receiving surface.

5. An in vivo image pickup device according to Claim 1, wherein the image pickup unit comprises a light source comprising at least one light-emitting element that emits light with a specific wavelength or white light.

6. An in vivo image pickup device according to Claim 5, wherein the light source comprises the light emitting elements that emit red, green, and blue light, and is controlled to sequentially emit one type of light every frame.

7. An in vivo image pickup device according to Claim 3, wherein the defect correcting circuit corrects the defective pixel based on positional information of the defective pixel stored in the storing circuit in advance.

8. An in vivo image pickup device according to Claim 3, wherein the defect correcting circuit detects the position of the defective pixel every starting timing of the device.

9. An in vivo image pickup device according to Claim 2, wherein the defect correcting circuit detects the position of the defective pixel every image pickup operation of the subject.

10. An in vivo image pickup device according to Claim 3, wherein the defect correcting circuit detects the position of the defective pixel every predetermined period.

11. An in vivo image pickup device according to Claim 2, wherein the detecting circuit detects the defective pixel based on the image signal from the pixel on the same line as that of a target pixel.

12. An in vivo image pickup device according to Claim 2, wherein the detecting circuit detects the target pixel as the defective pixel, when the image signal of the target pixel is higher than a maximum value of the image signals of a plurality of neighborhood pixels having the same color of the target pixel by a first threshold or more, or is lower than a minimum value by a second threshold or more.

13. An in vivo image pickup device according to Claim 2, wherein the detecting circuit detects the target pixel as the defective pixel, when the image signal of the target pixel is higher than an average of the image signals of a plurality of neighborhood pixels having the same color of the target pixel by a third threshold or more, or is lower than the average by a fourth threshold or more.

14. An in vivo image pickup device according to Claim 2, wherein the detecting circuit detects the defective pixel based on the image signals excluding image signals having a maximum value and a minimum value of a plurality of neighborhood pixels having the same color as that of the image signal of the target pixel.

15. An in vivo image pickup device according to Claim 2, wherein the detecting circuit detects the defect of the target pixel based on the pixel signal of the pixel having a color different from that of the target pixel.

16. An in vivo image pickup device according to Claim 2, wherein the correcting circuit corrects the image signal of the defective pixel by using the pixel on the same line as that of the defective pixel.

17. An in vivo image pickup device according to Claim 2, wherein the correcting circuit replaces the image signal of the defective pixel with the image signal of the most neighborhood pixel having the same color as that of the defective pixel.

18. An in vivo image pickup device according to Claim 2, wherein the correcting circuit obtains the image signal of the defective pixel with the linear interpolation of the image signal of the neighborhood pixel having the same color as that of the defective pixel.

19. An in vivo image pickup device according to Claim 6, further comprising:

combining means that combines the image signals of three frames obtained by sequentially emitting red, green, and blue light of the light source to color image signals corresponding one color image, wherein the compressing circuit compresses the image signal outputted by the combining means.

20. An in vivo image pickup device according to Claim 6, wherein the compressing circuit independently compresses the image signals of three frames obtained by sequentially emitting red, green, and blue light of the light source.

21. An in vivo image pickup system, comprising:

an in vivo image pickup device that is inserted in the body cavity and generates and sends an image signal of a subject, comprising an image pickup unit that has a plurality of pixels with arrays on a light receiving surface thereof and converts a subject image formed on the light receiving surface into the image signal, a defect correcting circuit that corrects the image signal of a defective pixel of the image pickup unit, a compressing circuit that compresses the image signal from the defect correcting circuit, and a sending circuit that sends the compressed image signal; and

a reception processing device that is separately arranged from the in vivo image pickup device and receives and processes the image signal sent from the in vivo image pickup device,

wherein the reception processing device comprises:

a receiving circuit that receives the image signal sent from the in vivo image pickup device; and

a signal processing circuit that performs predetermined signal processing of the image signal received by the receiving circuit.

# FIG.1

*1* : IN VIVO IMAGE PICKUP DEVICE

*2* : RECEPTION PROCESSING DEVICE

EP 1 618 833 A1

# FIG.2

EP 1 618 833 A1

*1a* : IN VIVO IMAGE PICKUP DEVICE

*11a* : IMAGE PICKUP UNIT        *12a* : DEFECT CORRECTING CIRCUIT        *13a* : COMPRESSING CIRCUIT

| *110* | *111* | | *121* | *122* | *123* | | *131* | *132* | | *14* |
|---|---|---|---|---|---|---|---|---|---|---|
| COLOR FILTERS | IMAGE PICKUP ELEMENT | | DETECTING CIRCUIT | STORING CIRCUIT | CORRECTING CIRCUIT | | PRE-PROCESSING CIRCUIT | ENCODING CIRCUIT | | SENDING CIRCUIT |

WHITE LIGHT SOURCE

*112*

# FIG.3

1002

| R | G r | (R) | G r | R | G r |
| G b | B | G b | B | G b | B |
| (R) | G r | (R) | G r | (R) | G r |
| G b | B | G b | B | G b | B |
| R | G r | (R) | G r | R | G r |

1003

1004

1001    1005

# FIG.4

1102

| R | G r | R | G r | R | G r |
| G b | B | (G b) | B | (G b) | B |
| R | G r | R | (G r) | R | G r |
| G b | B | (G b) | B | (G b) | B |
| R | G r | R | G r | R | G r |

1104

1101

1105

1103

# FIG.5

1001

X

A          1003

max

min

B          1002

# FIG.6

# FIG.7

12b : DEFECT CORRECTING
CIRCUIT

221 222 223

| DETECTING CIRCUIT | STORING CIRCUIT | CORRECTING CIRCUIT |

# FIG.8

# FIG.9

# FIG.10

# FIG.11

# FIG.12

# FIG.13

1c : IN VIVO IMAGE PICKUP DEVICE

11c : IMAGE PICKUP UNIT    12c : DEFECT CORRECTING CIRCUIT    13c : COMPRESSING CIRCUIT

| 311 | | 322 | 323 | 331 | 132 | 14 |
|---|---|---|---|---|---|---|
| IMAGE PICKUP ELEMENT | | STORING CIRCUIT | CORRECTING CIRCUIT | PRE-PROCESSING CIRCUIT | ENCODING CIRCUIT | SENDING CIRCUIT |

| LIGHT SOURCE R | LIGHT SOURCE G | LIGHT SOURCE B |
|---|---|---|

312

EP 1 618 833 A1

# FIG.14

# FIG.15

# FIG.16

FIG.17

# FIG.18

R G B R G B R G B

Y, Cb, Cr
Y, Cb, Cr
Y, Cb, Cr

# FIG.19

12d : DEFECT CORRECTING CIRCUIT

13d : COMPRESSING CIRCUIT

421

DETECTING CIRCUIT

423

CORRECTING CIRCUIT

432

ENCODING CIRCUIT

# FIG.20

4001

| R | R | R | R | R | R |
|---|---|---|---|---|---|
| R | R | R | R | R | R |
| R | R | R | R | R | R |
| R | R | R | R | R | R |
| R | R | R | R | R | R |

4002

4003  4004  4005

4006

# FIG.21

4001

| R | R | R | R | R | R |
|---|---|---|---|---|---|
| R | R | R | R | R | R |
| R | R | R | R | R | R |
| R | R | R | R | R | R |
| R | R | R | R | R | R |

4002

4003  4004  4005

4006

# FIG.22

| R | R | R | R | R | R |
|---|---|---|---|---|---|
| R | R | R | R | R | R |
| R | R | R | R | R | R |
| R | R | R | R | R | R |
| R | R | R | R | R | R |

| G | G | G | G | G | G |
|---|---|---|---|---|---|
| G | G | G | G | G | G |
| G | G | G | G | G | G |
| G | G | G | G | G | G |
| G | G | G | G | G | G |

| B | B | B | B | B | B |
|---|---|---|---|---|---|
| B | B | B | B | B | B |
| B | B | B | B | B | B |
| B | B | B | B | B | B |
| B | B | B | B | B | B |

# FIG.23

*1e* : IN VIVO IMAGE PICKUP DEVICE

*11e* : IMAGE PICKUP UNIT

*12c* : DEFECT CORRECTING CIRCUIT

*13d* : COMPRESSING CIRCUIT

*311*

*322*    *323*

*432*    *14*

| IMAGE PICKUP ELEMENT |
| SINGLE-WAVELENGTH LIGHT SOURCE |

STORING CIRCUIT

CORRECTING CIRCUIT

ENCODING CIRCUIT

SENDING CIRCUIT

*511*

# FIG.24
## (RELATED ART)

901 : IN VIVO IMAGE PICKUP DEVICE

902 : DISPLAY SYSTEM

| 903 | 904 | 905 |
|---|---|---|
| IMAGE PICKUP MEANS | COMPRESSING MEANS | SENDING MEANS |

SEND

| 906 | 907 | 908 |
|---|---|---|
| RECEIVING MEANS | DECOMPRESSING MEANS | DISPLAY MEANS |

EP 1 618 833 A1

European Patent
Office

**EUROPEAN SEARCH REPORT**

Application Number

EP 05 01 5464

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | WO 03/010967 A (GIVEN IMAGING LTD; GLUKHOVSKY, ARKADY; AVNI, DOV; MERON, GAVRIEL) 6 February 2003 (2003-02-06) <br> * page 2, line 31 - page 7, line 30 * <br> * figure 1 * <br>----- | 1-21 | A61B1/05 <br> H04N5/217 |
| Y | EP 1 250 000 A (AGILENT TECHNOLOGIES, INC.) 16 October 2002 (2002-10-16) <br><br> * paragraphs [0003], [0004] * <br> * paragraph [0008] * <br> * paragraphs [0013], [0014] * <br> * paragraphs [0018], [0024], [0025] * <br>----- | 1-7,9, 16,17, 19-21 | |
| Y | US 6 683 643 B1 (TAKAYAMA JUN ET AL) 27 January 2004 (2004-01-27) <br> * column 2, lines 24-32 * <br> * column 11, lines 51,52 * <br> * column 12, lines 26-46 * <br>----- | 8,10 | |
| Y | EP 1 003 332 A (VISION GROUP PLC) 24 May 2000 (2000-05-24) <br> * paragraphs [0001] - [0007] * <br> * paragraph [0015] * <br> * paragraphs [0031] - [0036] * <br>----- | 11,12, 14,18 | TECHNICAL FIELDS SEARCHED (Int.Cl.7) <br><br> A61B <br> H04N |
| Y | EP 1 401 196 A (AGILENT TECHNOLOGIES, INC) 24 March 2004 (2004-03-24) <br> * paragraph [0005] * <br> * paragraph [0008] * <br>----- | 13,15 | |
| A | US 6 509 927 B1 (PRATER JAMES S ET AL) 21 January 2003 (2003-01-21) <br> * column 1, line 56 - column 2, line 9 * <br> * column 7, line 40 - column 8, line 33 * <br> * column 9, lines 28-38 * <br>----- | 1,2,9, 11,16,17 | |

-/--

The present search report has been drawn up for all claims

| Place of search | Date of completion of the search | Examiner |
|---|---|---|
| Berlin | 12 October 2005 | Völlinger, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................

& : member of the same patent family, corresponding document

EPO FORM 1503 03.82 (P04C01)

**European Patent Office**

## EUROPEAN SEARCH REPORT

Application Number

EP 05 01 5464

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| P,A | US 2005/049461 A1 (HONDA TAKEMITSU ET AL) 3 March 2005 (2005-03-03) * paragraphs [0015], [0027] * * paragraphs [0064] - [0068] * * paragraph [0096] * * paragraphs [0160] - [0176] * | 1 | |
| A | PATENT ABSTRACTS OF JAPAN vol. 017, no. 393 (E-1402), 22 July 1993 (1993-07-22) & JP 05 068209 A (TOSHIBA CORP), 19 March 1993 (1993-03-19) * abstract * | 1 | |
| | | | TECHNICAL FIELDS SEARCHED (Int.Cl.7) |

The present search report has been drawn up for all claims

| Place of search | Date of completion of the search | Examiner |
|---|---|---|
| Berlin | 12 October 2005 | Völlinger, M |

EPO FORM 1503 03.82 (P04C01)

## ANNEX TO THE EUROPEAN SEARCH REPORT
## ON EUROPEAN PATENT APPLICATION NO.

EP 05 01 5464

This annex lists the patent family members relating to the patent documents cited in the above-mentioned European search report.
The members are as contained in the European Patent Office EDP file on
The European Patent Office is in no way liable for these particulars which are merely given for the purpose of information.

12-10-2005

| Patent document cited in search report | | Publication date | Patent family member(s) | | Publication date |
|---|---|---|---|---|---|
| WO 03010967 | A | 06-02-2003 | CN | 1586082 A | 23-02-2005 |
| | | | EP | 1433325 A1 | 30-06-2004 |
| | | | JP | 2004536644 T | 09-12-2004 |
| EP 1250000 | A | 16-10-2002 | JP | 2003009004 A | 10-01-2003 |
| | | | US | 2002149683 A1 | 17-10-2002 |
| US 6683643 | B1 | 27-01-2004 | JP | 10322603 A | 04-12-1998 |
| EP 1003332 | A | 24-05-2000 | GB | 2345607 A | 12-07-2000 |
| EP 1401196 | A | 24-03-2004 | JP | 2004112802 A | 08-04-2004 |
| | | | US | 2004051798 A1 | 18-03-2004 |
| US 6509927 | B1 | 21-01-2003 | JP | 8237550 A | 13-09-1996 |
| US 2005049461 | A1 | 03-03-2005 | WO | 2004112593 A1 | 29-12-2004 |
| JP 05068209 | A | 19-03-1993 | NONE | | |

EPO FORM P0459

For more details about this annex : see Official Journal of the European Patent Office, No. 12/82